(19) Europäisches Patentamt
European Patent Office
Office européen des brevets

(11) Veröffentlichungsnummer: **0 257 411 B1**

(12) # EUROPÄISCHE PATENTSCHRIFT

(45) Veröffentlichungstag der Patentschrift: **25.11.92**

(21) Anmeldenummer: **87111487.2**

(22) Anmeldetag: **08.08.87**

(51) Int. Cl.5: **C07C 209/68**, C07C 213/08, C07C 211/20, C07C 211/40, C07D 295/02, C07D 295/08, C07C 215/08, C07C 47/02, C07C 47/19, C07B 53/00, B01J 31/24

(54) **Verfahren zur Herstellung von optisch aktiven Enaminen oder von den entsprechenden Aldehyden.**

(30) Priorität: **21.08.86 CH 3352/86**

(43) Veröffentlichungstag der Anmeldung:
**02.03.88 Patentblatt 88/09**

(45) Bekanntmachung des Hinweises auf die Patenterteilung:
**25.11.92 Patentblatt 92/48**

(84) Benannte Vertragsstaaten:
**AT BE CH DE FR GB IT LI NL**

(56) Entgegenhaltungen:
**EP-A- 0 068 506**
**EP-A- 0 135 392**
**EP-A- 0 156 607**
**EP-A- 0 170 470**
**EP-A- 0 235 450**

**J. Am. Chem. Soc., vol. 106(18), 1984, S. 5208-5217**

(73) Patentinhaber: **F. HOFFMANN-LA ROCHE AG**
**Postfach 3255**
**CH-4002 Basel(CH)**

(72) Erfinder: **Heiser, Bernd, Dr.**
**Kalchmattweg 13A**
**W-7854 Inzlingen(DE)**
Erfinder: **Stoller, Hansjörg, Dr.**
**Niederbergstrasse 21**
**CH-4153 Reinach(CH)**

(74) Vertreter: **Cottong, Norbert A. et al**
**Grenzacherstrasse 124 Postfach 3255**
**CH-4002 Basel(CH)**

EP 0 257 411 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung von chiralen Enaminen der allgemeinen Formel

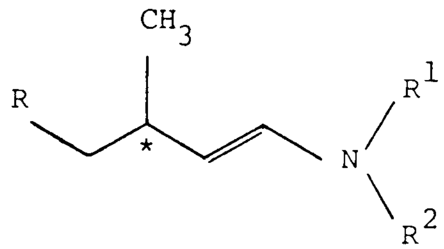

I

worin R einen Rest der Formel

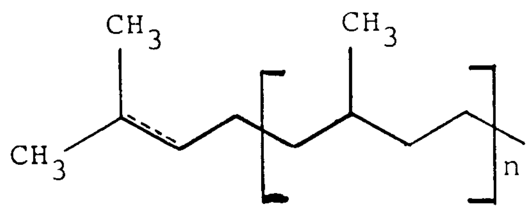

a)

oder

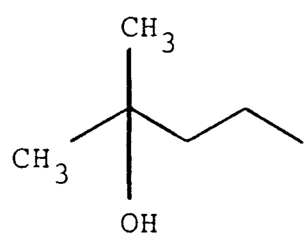

b)

bedeutet, worin n eine Zahl 0, 1 oder 2 und die gestrichelte Linie eine fakultative zusätzliche Bindung darstellen und $R^1$ und $R^2$ niederes Alkyl oder Cycloalkyl oder zusammen mit dem Stickstoffatom einen 5- oder 6-gliedrigen heterocyclischen Ring darstellen,
sowie von den entsprechenden Aldehyden, durch Isomerisierung der E- oder Z-Form einer Verbindung der allgemeinen Formel

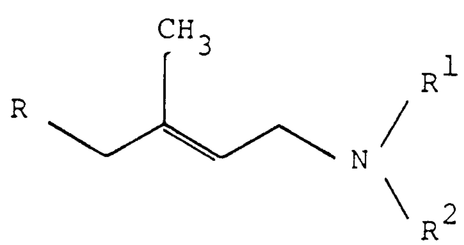

II

worin R, $R^1$ und $R^2$ die obige Bedeutung haben. mit einem Rhodium-diphosphinkomplex der allgemeinen Formel

$[Rh(L^1)(L^2)]^+ X^-$ III

worin $L^1$ einen chiralen Diphosphinliganden. $L^2$ ein bis-Olefin und $X^-$ ein nicht komplexierendes Anion darstellen,

dadurch gekennzeichnet, dass man die Isomerisierung unter Zusatz eines achiralen Triarylphosphins durchführt und, gewünschtenfalls, eine so erhaltene Verbindung der Formel I hydrolysiert.

Die Erfindung betrifft ebenfalls die aus einem Rhodium-diphosphinkomplex der allgemeinen Formel III und einem achiralen Triarylphosphin erhältlichen Komplexe.

Die erfindungsgemäss erhaltenen Verbindungen der Formel I sowie die entsprechenden Aldehyde sind bekannte Zwischenprodukte in der Herstellung von u.a. natürlichem Vitamin E, natürlichem Vitamin $K_1$ und auch Riechstoffen. Die Hydrolyse der Verbindungen der Formel I zu den entsprechenden Aldehyden kann in bekannter Weise erfolgen.

Die als Ausgangsmaterial verwendeten Verbindungen der allgemeinen Formel II sind bekannte Verbindungen oder Analoge bekannter Verbindungen, welche leicht in zur Herstellung der bekannten Verbindungen analoger Weise hergestellt werden können. Derartige Verbindungen sind beispielsweise bekannt aus EP 68 506.

Aus EP 68 506 ist auch die Isomerisierung derartiger Allylamine zu chiralen Enaminen unter Verwendung von Rhodium-phosphinkomplexen bekannt. Aus EP 135 392 und EP 156 607 sind weiterhin gegenüber EP 68 506 verbesserte Rhodium-phosphinkomplexe für derartige Isomerisierungen bekannt.

Die Rhodium-diphosphinkomplexe der Formel III sind bekannte Komplexe und können nach an sich bekannten Verfahren hergestellt werden. Als chirale Diphosphinliganden kommen im Rahmen der vorliegenden Erfindung insbesondere in Frage optisch aktive, in (R)- oder (S)-Form vorliegende Verbindungen der allgemeinen Formel

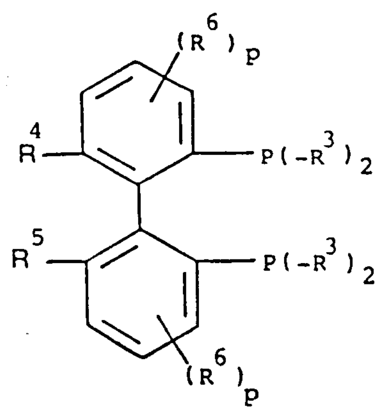

IV

worin $R^3$ Phenyl, $R^4$ und $R^5$, welche gleich oder verschiedenen sein können, Wasserstoff, niederes Alkyl, niederes Alkoxy, Di-niederes Alkylamino, geschütztes Hydroxymethyl oder $R^4$ und $R^5$ zusammen die Gruppen $-(CH_2-)_m$, $-CH_2-O-CH_2-$,

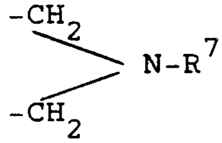

oder

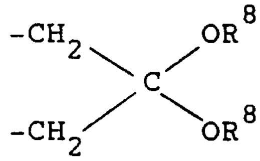

bedeuten, wobei m eine Zahl 3 bis 5, $R^7$ niederes Alkyl, Phenyl oder Benzyl und $R^8$ niederes Alkyl oder beide $R^8$ zusammen niederes Alkylen darstellen, $R^6$ Methyl, niederes Alkoxy, Di-niederes Alkylamino oder Fluor und p die Zahl 0, 1, 2 oder 3 bedeuten,

oder auch optisch aktive, in (R)- oder (S)-Form vorliegende Verbindungen der allgemeinen Formel

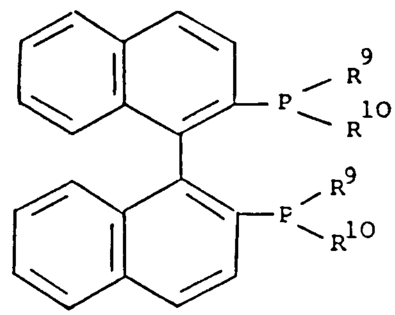

V

worin $R^9$ und $R^{10}$ Phenyl oder Cyclohexyl bedeuten und die Naphthalinringe gegebenenfalls noch in ortho-Stel-lung mit Methyl, Aethyl, Halogen, Di-niederes Alkylamino oder niederes Alkoxy substituiert sein können.

Der Ausdruck "niederes Alkyl" bedeutet insbesondere geradkettige oder verzweigte Alkylgruppen mit 1-4 Kohlenstoffatomen wie Methyl, Aethyl, Propyl, Isopropyl, n-Butyl, Isobutyl und tert.-Butyl. Der Ausdruck "Cycloalkyl" bedeutet insbesondere Cyclopropyl, Clyclopentyl, Cyclohexyl und dergleichen. Falls die Substituenten $R^1$ und $R^2$ zusammen mit dem Stickstoffatom einen heterocyclischen Ring darstellen, ist dies vorzugsweise ein Pyrrolidin- oder Piperidinring. In einem derartigen Ring können jedoch auch noch ein weiteres Sauerstoffatom oder ein gegebenenfalls alkyliertes ($C_1$-$C_4$) oder benzyliertes Stickstoffatom enthalten sein, z.B. der Morpholin- oder Piperazinring. Der Ausdruck "nicht komplexierendes Anion" bedeutet insbesondere $BF_4^-$, $PF_6^-$, $ClO_4^-$, B(Phenyl)$_4^-$, $PCl_6^-$ usw.

Als Schutzgruppen für die Hydroxymethylgruppen kommen im Rahmen der vorliegenden Erfindung insbesondere in Frage die üblichen, Aether-bildenden Gruppen wie z.B. Benzyl, Methyl, tert.-Butyl, Allyl, Methoxymethoxy und dergleichen, sowie auch Silyläther-bildende Gruppen, wie z.B. Trimethylsilyl, tert.Butyldimethylsilyl und dergleichen.

Die erwähnten Phenyl- und Benzylreste können, im Rahmen der vorliegenden Erfindung, sowohl unsubstituiert als auch in ortho-, meta- oder para-Stellung oder auch mehrfach substituiert sein. als Substituenten kommen hier in Frage niedere Alkyl- oder niedere Alkoxygruppen, vorzugsweise Methyl- oder Methoxygruppen, oder auch Di- niederes Alkylamino, vorzugsweise Dimethylaminogruppen, sowie Fluor. Die Ausdrücke "niederes Alkoxy", "Di-niederes Alkylamino" und "niederes Alkoxycarbonyl" bedeuten Gruppen, in denen der Alkylrest die vorhergehend erwähnte Bedeutung haben kann.

Als Beispiele von bis-Olefinen können genannt werden 1,5-Cyclooctadien, 1,5-Hexadien, Bicyclo[2.2.1]-hepta-2,5-dien usw.

Die Verbindungen der allgemeinen Formeln IV und V sind bekannte Verbindungen. Besonders bevorzugte Verbindungen der Formel IV sind solche, worin $R^3$ unsubstituiertes oder Methyl oder Fluor substituiertes Phenyl, $R^4$ und $R^5$ gleich sind und niederes Alkyl oder zusammen die Gruppe $-CH_2-O-CH_2-$, p die Zahl O oder 1 und $R^6$ Methyl, Fluor oder Di-niederes Alkylamino bedeuten. Sofern p die Zahl 1 darstellt, befindet sich der Substituent $R^6$ vorzugsweise in m-Stellung zum Phosphor.

Als Beispiele von bevorzugten Verbindungen der Formel IV können folgende genannt werden:

(R)- oder (S)-(6,6′-Dimethyl-2-2′-biphenylylen)bis(diphenylphosphin),
(R)- oder (S)-(4,4′,6,6′-Tetramethyl-2,2′-biphenylylen)bis(disphenylphosphin),
(R)- oder (S)-3,3′6,6′-Tetramethyl-2,2′-biphenylylen)bis(diphenylphosphin),
(R)- oder (S)-(4,4′-Bis(dimethylamino)-6,6′-dimethyl-2,2′-biphenylylen)bis(disphenylphosphin),
(R)- oder (S)-(4,4′-Difluor-6,6′-dimethyl-2,2′-biphenylylen)bis(diphenylphosphin),
(R)- oder (S)-(6,6′-Dimethyl-2,2′-biphenylylen)bis(di-p-tolylphosphin),
(R)- oder (S)-(6,6′-Dimethyl-2,2′-biphenylylen)bis(di-o-tolylphosphine),
(R)- oder (S)-(6,6′-Dimethyl-2,2′-biphenylylen)bis(di-m-fluorphenylphosphin),
(R)- oder (S)-1,11-Bis(diphenylphosphino)-5,7-dihydrodibenz[c,e]oxepin.

Ganz besonders bevorzugt ist hierbei (R)-6,6′-Dimethyl-2,2′-biphenylylen)bis(diphenylphosphin).

Eine besonders bevorzugte Verbindung der Formel V ist (R)-(1,1′-Binaphthyl)-2,2′-diylbis-(diphenylphosphin).

Als achirale Triarylphosphine kommen im Rahmen der vorliegenden Erfindung insbesondere in Frage

solche, in denen die drei Arylreste identisch sind, vorzugsweise Triphenylphosphin.

Die erfindungsgemässe Isomerisierung kann in geeigneten, unter den Reaktionsbedingungen inerten organischen Lösungsmitteln durchgeführt werden. Als solche können insbesondere genannt werden niedere Alkanole wie z.B. Methanol oder Aethanol, aromatische Kohlenwasserstoffe wie Benzol oder Toluol, cyclische Aether wie Tetrahydrofuran oder Dioxan, Ester wie z.B. Essigester oder auch Gemische hiervon und dergleichen.

Wie erwähnt erfolgt die erfindungsgemässe Isomerisierung mittels eines Komplexes der Formel III in Gegenwart eines achiralen Triarylphosphins. Hierbei kann so vorgegangen werden, dass das zu isomerisierende Substrat zusammen mit dem Komplex der Formel III und dem achiralen Triarylphosphin umgesetzt wird. Andererseits kann auch zuerst der Komplex der Formel III mit einem achiralen Triarylphosphin umgesetzt werden und das erhaltene Produkt dann, gegebenenfalls nach dessen Isolierung, mit dem Substrat der Formel II umgesetzt werden.

Die Isomerisierung kann zweckmässig in einem inerten, organischen Lösungsmittel und bei einer Temperatur von etwa Raumtemperatur bis etwa 130 °C durchgeführt werden. Diese Reaktion erfolgt vorzugsweise bei erhöhter Temperatur, d.h. je nach verwendetem Lösungsmittel entweder bei Rückflusstemperatur des Reaktionsgemisches oder in einem geschlossenen Gefäss, unter Druck.

Das molare Verhältnis von Komplex der Formel III zu achiralem Triarylphosphin beträgt zweckmässig 1 zu 2 bis 10, vorzugsweise 1 zu 2 bis 4.

Durch das erfindungsgemässe Verfahren, d.h. durch die Isomerisierung der Verbindungen der Formel II mit einem Komplex der Formel III in Gegenwart eines Triarylphosphins kann die Recylisierung des Katalysators wesentlich vereinfacht und verbilligt werden. Gleichzeitig wird durch die Zugabe des billigen und leicht zugänglichen Triarylphosphins die Lebensdauer und die Aktivität des Katalysators erheblich vergrössert.

## Beispiel 1

In ein 25 ml Schlenkrohr werden 1 g (4,78 mMol) N,N-Diäthylnerylamin, 20,3 mg (0,024 mMol) [$\eta^4$-1,5-Cyclooctadien][(R)-(6,6′-dimethyl-2,2′ -biphenylylen)bis(diphenylphosphin)]rhodium(I) -tetrafluoroborat, 18.8 mg (0,072 mMol) Triphenylphosphin und 5 ml absolutes Tetrahydrofuran eingefüllt. Das Schlenkrohr wird dicht verschlossen, durch dreimaliges Ausfrieren/Evakuieren entgast, unter Stickstoff gesetzt und in einem Oelbad 16 Stunden bei 100 °C erhitzt. Danach wird das Tetrahydrofuran im Hochvakum (0,2 mbar) abgezogen, der Katalysator mit 5 ml Pentan ausgefällt und die überstehende schwach rot gefärbte Lösung in einen Kolben pipettiert. Das verbleibende rotbraune Pulver wird zweimal mit je 5 ml Pentan gewaschen. Die vereinigten Pentanlösungen werden eingeengt und im Kugelrohrofen (ca. 100 °C/0,5 mbar) destilliert. Es werden 0,95 g farbloses Oel erhalten. Das Destillat wird bei 0 °C mit dem dreifachen Volumen an 30%iger wässriger Essigsäure versetzt, 15 Minuten kräftig gerührt, mit ca. 5 ml Pentan überschichtet und weitere 15 Minuten bei Raumtemperatur gerührt. Nach Phasentrennung wird noch zweimal mit Pentan extrahiert. Die vereinigten organischen Phasen werden nacheinander mit gesättigter $NaHCO_3$-Lösung, Wasser, gesättigter NaCl-Lösung gewaschen, über $Na_2SO_4$ getrocknet, filtriert und eingedampft. Nach Kugelrohrdestillation bei 100-110 °C/15 mbar erhält man 0,6 g (R)-Citronellal (97% e.e. nach gaschromatographischer Methode).

Zum Katalysatorrückstand werden ca. 2 mg vom obigen Rhodium-Komplex und 2 mg Triphenylphosphin gegeben und wie vorhergehend beschrieben mit der gleichen Menge N,N-Diäthylnerylamin umgesetzt. Man erhält (R)-Citronellal in praktisch gleicher Ausbeute mit einem e.e. Gehalt von 96,5%. Nochmalige Recyclisierungen liefern 0,58 g bzw. 0,52 g (R)-Citronellal mit e.e. Gehalten von 96,2% bzw. 96,8%.

## Beispiel 2

In zu Beispiel 1 analoger Weise wird N,N-Diäthylnerylamin isomerisiert, jedoch unter Verwendung von-[$\eta^4$-1,5-Cyclooctadien][(R)-(1,1′-binaphthyl)-2,2′-diylbis(diphenylphosphin)]rhodium(I)-tetrafluoroborat. Man erhält 0,61 g (R)-Citronellal mit 97,2% e.e. nach der gaschromatographischen Methode.
Die ebenfalls in zu Beispiel 1 analoger Weise durchgeführten Recyclisierungen des Katalysators führen zu 0,59 g, 0,63 g bzw. 0,57 g (R)-Citronellal mit 97,5% e.e., 98,7% e.e. bzw. 98,1% e.e.

## Patentansprüche

**1.** Verfahren zur Herstellung von chiralen Enaminen der allgemeinen Formel

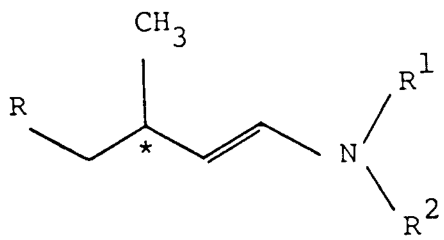

I

worin R oder einen Rest der Formel

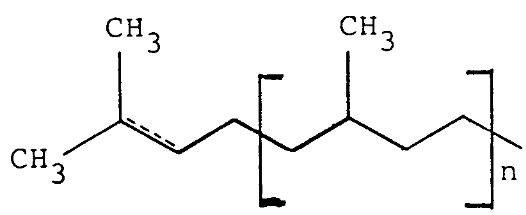

a)

oder

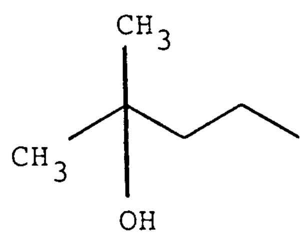

b)

bedeutet, worin n eine Zahl 0, 1 oder 2 und die gestrichelte Linie eine fakultative zusätzliche Bindung darstellen und $R^1$ und $R^2$ niederes Alkyl oder Cycloalkyl oder zusammen mit dem Stickstoffatom einen 5- oder 6-gliedrigen heterocyclischen Ring darstellen,
sowie von den entsprechenden Aldehyden, durch Isomerisierung der E- oder Z-Form einer Verbindung der allgemeinen Formel

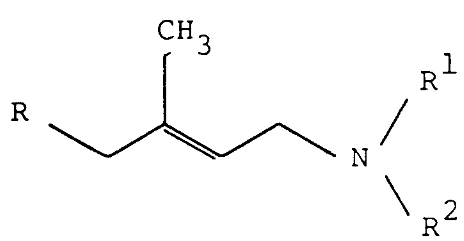

II

worin R, $R^1$ und $R^2$ die obige Bedeutung haben, mit einem Rhodium-diphosphinkomplex der allgemeinen Formel

$[Rh(L^1)(L^2)]^+ X^-$ III

worin $L^1$ einen chiralen Diphosphinliganden, $L^2$ ein bis-Olefin und $X^-$ ein nicht komplexierendes Anion darstellen,
dadurch gekennzeichnet, dass man die Isomerisierung unter Zusatz eines achiralen Triarylphosphins durchführt und, gewünschtenfalls, eine so erhaltene Verbindung der Formel I hydrolysiert.

**2.** Verfahren gemäss Anspruch 1, dadurch gekennzeichnet, dass als chiraler Diphosphinligand $L^1$, in einer Verbindung der Formel III, eine optisch aktive, in (R)- oder (S)-Form vorliegende Verbindung verwendet

wird der allgemeinen Formel

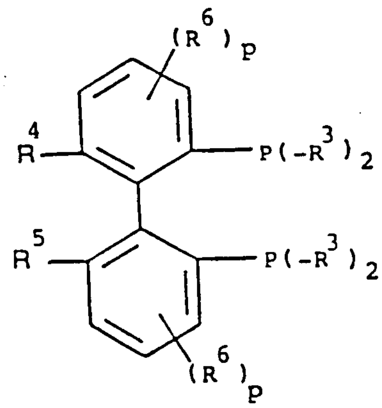

IV

worin $R^3$ Phenyl, $R^4$ und $R^5$, welche gleich oder verschiedenen sein können, Wasserstoff, niederes Alkyl, niederes Alkoxy, Di-niederes Alkylamino, geschütztes Hydroxymethyl oder $R^4$ und $R^5$ zusammen die Gruppen
$-(-CH_2-)_m$, $-CH_2-O-CH_2-$,

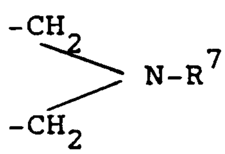

oder

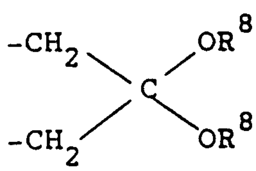

bedeuten, wobei m eine Zahl 3 bis 5, $R^7$ niederes Alkyl, Phenyl oder Benzyl und $R^8$ niederes Alkyl oder beide $R^8$ zusammen niederes Alkylen darstellen, $R^6$ Methyl, niederes Alkoxy, Di-niederes Alkylamino oder Fluor und p die Zahl 0, 1, 2 oder 3 bedeuten,
oder auch eine optisch aktive, in (R)- oder (S)-Form vor-liegende Verbindung der allgemeinen Formel

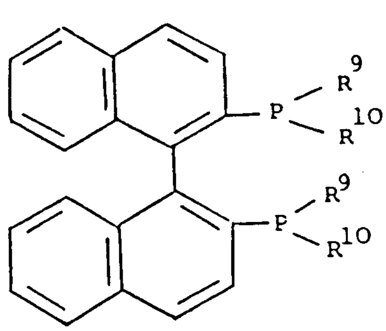

V

worin $R^9$ und $R^{10}$ Phenyl oder Cyclohexyl bedeuten und die Naphthalinringe gegebenenfalls noch in ortho-Stellung mit Methyl, Aethyl, Halogen, Di-niederes Alkylamino oder niederes Alkoxy substituiert sein können.

3. Verfahren gemäss Anspruch 1 oder 2, dadurch gekennzeichnet, dass als chiraler Diphosphinligand $L^1$, in einer Verbindung der Formel III, eine Verbindung der Formel IV verwendet wird, worin $R^3$ unsubstituiertes oder Methyl oder Fluor substituiertes Phenyl, $R^4$ und $R^5$ gleich sind und niederes Alkyl oder zusammen die Gruppe $-CH_2-O-CH_2-$, p die Zahl 0 oder 1 und $R^6$ Methyl, Fluor oder Di-niederes Alkylamino bedeuten.

4. Verfahren gemäss einem der Ansprüche 1 bis 3, dadurch gekennzeichnet, dass als chiraler Diphosphinligand $L^1$, in einer Verbindung der Formel III, (R)-(6,6′-Dimethyl-2,2′-biphenylylen)bis-(diphenylphosphin) verwendet wird.

5. Verfahren gemäss Anspruch 1 oder 2, dadurch gekennzeichnet, dass als chiraler Diphosphinligand $L^1$, in einer Verbindung der Formel III, (R)-(1,1′-Binaphtyl-2,2′-diylbis(diphenylphosphin) verwendet wird.

6. Verfahren gemäss einem der Ansprüche 1 bis 5, dadurch gekennzeichnet, dass man ein Ausgangsmaterial der Formel II verwendet, worin $R^1$ und $R^2$ Aethyl bedeuten und R einen Rest der Formel a) darstellt, worin n gleich 0 ist.

7. Verfahren gemäss einem der Ansprüche 1 bis 6, dadurch gekennzeichnet, dass man als achirales Triarylphosphin eines verwendet, worin die drei Arylreste identisch sind.

8. Verfahren gemäss Anspruch 7, dadurch gekennzeichnet, dass man als achirales Triarylphosphin Triphenylphosphin verwendet.

9. Komplex aus einem Rhodium-diphosphinkomplex der Formel III und einem achiralen Triarylphosphin, vorzugsweise Triphenylphosphin.

**Claims**

1. A process for the manufacture of chiral enamines of the general formula

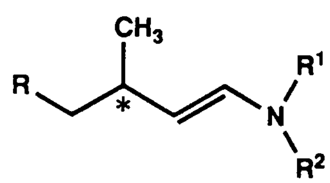

I

wherein R signifies a residue of the formula

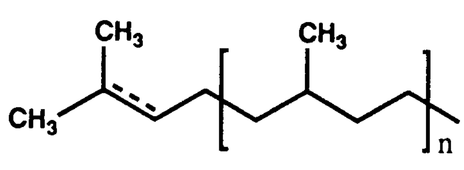

a)

or

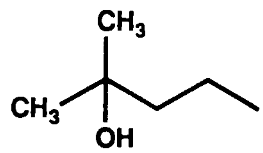

b)

wherein n represent a number 0, 1 or 2 and the dotted line represent a facultative additional bond and $R^1$ and $R^2$ represent lower alkyl or cycloalkyl or together with the nitrogen atom represent a 5- or 6-membered heterocyclic ring,
and of the corresponding aldehydes by isomerizing the E- or Z-form of a compound of the general formula

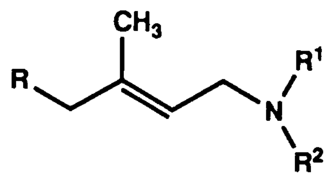

II

wherein R, $R^1$ and $R^2$ have the above significance, with a rhodium-diphosphine complex of the general formula

$[Rh(L^1)(L^2)]^+X^-$ III

wherein $L^1$ represents a chiral diphosphine ligand, $L^2$ represents a bis-olefin and X- represents a non-complexing anion,
characterized by carrying out the isomerization with the addition of an achiral triarylphosphine and, if desired, hydrolyzing a thus-obtained compound of formula I.

**2.** A process in accordance with claim 1, characterized in that as the chiral diphosphine ligand $L^1$ in a compound of formula III there is used an optically active compound of the general formula which is present in (R)- or (S)-form

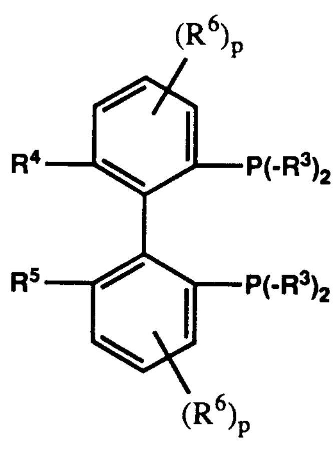

IV

wherein $R^3$ signifies phenyl, $R^4$ and $R^5$ which can be the same or different, signify hydrogen, lower alkyl, lower alkoxy, di-lower alkylamino, protected hydroxymethyl or $R^5$ and $R^6$ together signify the groups
$-(-CH_2-)_m$, $-CH_2-O-CH_2-$,

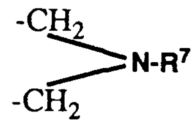

or

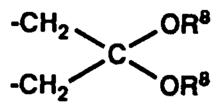

whereby m represents a number 3 to 5, $R^7$ represents lower alkyl, phenyl or benzyl and $R^8$ represents lower alkyl or the two $R^8$'s together represent lower alkylene, $R^6$ signifies methyl, lower alkoxy, di-lower alkylamino or fluorine and p signifies the number 0, 1, 2 or 3,
or an optically active compound of the general formula which is present in (R)- or (S)-form

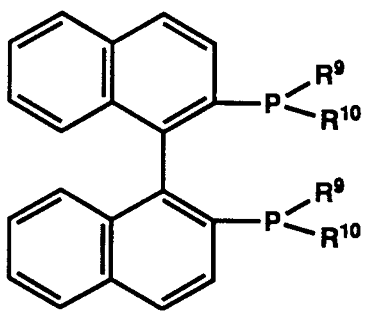

V

wherein $R^9$ and $R^{10}$ signify phenyl or cyclohexyl and the naphthalene rings can be optionally substituted in the ortho-position with methyl, ethyl, halogen, di-lower alkylamino or lower alkoxy.

**3.** A process in accordance with claim 1 or 2, characterized in that a compound of formula IV in which $R^3$ signifies unsubstituted phenyl or methyl- or fluoro-substituted phenyl, $R^4$ and $R^5$ are the same and signify lower alkyl or together signify the group $-CH_2-O-CH_2-$, p signifies the number 0 or 1 and $R^6$ signifies methyl, fluorine or di-lower alkylamino is used as the chiral diphosphine ligand $L^1$ in a compound of formula III.

**4.** A process in accordance with any one of claims 1 to 3, characterized in that (R)-(6,6'-dimethyl-2,2'-biphenylylene)bis(diphenylphosphine) is used as the chiral diphosphine ligand $L^1$ in a compound of formula III.

**5.** A process in accordance with claim 1 or 2, characterized in that (R)-(1,1'-binaphthyl)-2,2'-diylbis-(diphenylphosphine) is used as the chiral diphosphine ligand $L^1$ in a compound of formula III.

**6.** A process in accordance with any one of claims 1 to 5, characterized in that a starting material of formula II in which $R^1$ and $R^2$ signify ethyl and R represents a residue of formula a) in which n is 0 is used.

**7.** A process in accordance with any one of claims 1 to 6, characterized in that an achiral triarylphosphine in which the three aryl residues are identical is used as the achiral triarylphosphine.

**8.** A process in accordance with claim 7, characterized in that triphenylphosphine is used as the achiral triarylphosphine.

9. Complexes from a rhodium-diphosphine complex of formula III and an achiral triarylphosphine, preferably triphenylphosphine.

**Revendications**

1. Procédé de préparation d'énamines chirales de formule générale

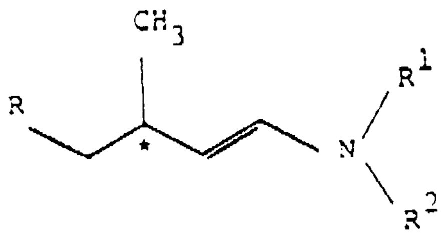

I

dans laquelle R représente un radical de formule

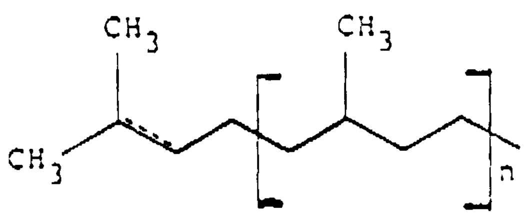

a)

ou

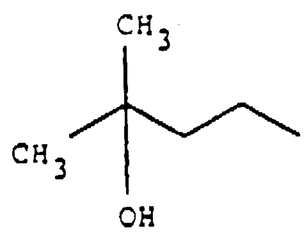

b)

où n représente le nombre 0, 1 ou 2 et la ligne pointillée représente une liaison supplémentaire facultative et $R^1$ et $R^2$ représentent un alcoyle inférieur ou un cycloalcoyle, ou représentent ensemble avec l'atome d'azote un noyau hétérocyclique à 5 ou 6 chaînons,
ainsi que des aldéhydes correspondants, par isomérisation de la forme E Ou Z d'un composé de formule générale

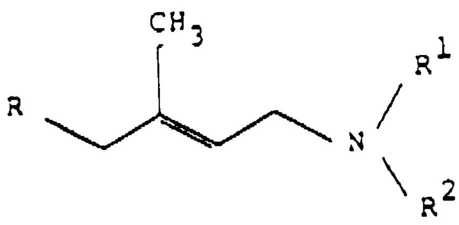

II

dans laquelle R, $R^1$ et $R^2$ ont la signification donnée ci-dessus,
avec un complexe de rhodium-diphosphine de formule générale

$[Rh(L^1)(L^2)]^+ X^-$ III

dans laquelle $L^1$ représente un coordinat de diphosphine chiral, $L^2$ une bis-oléfine et $X^-$ un anion non complexant,
caractérisé en ce qu'on effectue l'isomérisation en ajoutant une triarylphosphine achirale et, si on le désire, en hydrolysant un composé de formule I ainsi obtenu.

**2.** Procédé selon la revendication 1, caractérisé en ce qu'on utilise comme coordinat de diphosphine chiral $L^1$, dans un composé de formule III, un composé optiquement actif, se présentant sous forme (R) ou (S), de formule générale

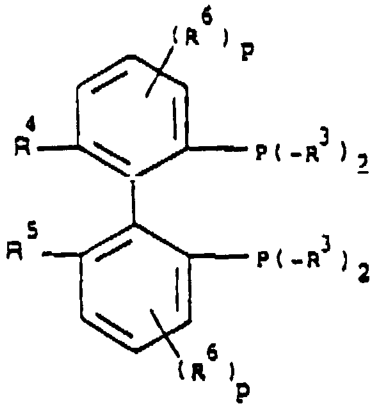

IV

dans laquelle $R^3$ représente un phényle, $R^4$ et $R^5$ , qui peuvent être identiques ou différents, représentent un hydrogène, un alcoyle inférieur, un alcoxy inférieur, un di-alcoyle inférieur-amino, un hydroxyméthyle protégé, ou $R^4$ et $R^5$ représentent ensemble les groupes
$-(-CH_2-)_m$, $-CH_2-O-CH_2-$,

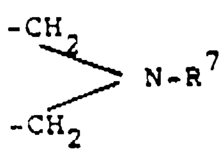

ou

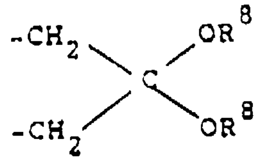

où m représente un nombre allant de 3 à 5, $R^7$ un alcoyle inférieur, un phényle ou un benzyle et $R^8$ un alcoyle inférieur, ou les deux $R^8$ représentent ensemble un alcoylène inférieur, $R^6$ représente un méthyle, un alcoxy inférieur, un di-alcoyle inférieur-amino ou un fluor et p représente le nombre 0, 1, 2 ou 3,
ou encore un composé optiquement actif, se présentant sous forme (R) ou (s), de formule générale

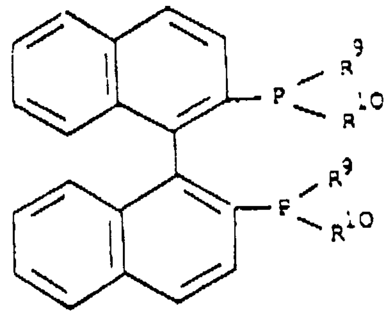

V

dans laquelle $R^9$ et $R^{10}$ représentent un phényle ou un cyclohexyle et les noyaux naphtalène peuvent le cas échéant être encore substitués en position ortho par un méthyle, un éthyle, un halogène, un di-alcoyle inférieur-amino ou un alcoxy inférieur.

**3.** Procédé selon la revendication 1 ou 2, caractérisé en ce qu'on utilise comme coordinat de diphosphine chiral $L^1$ , dans un composé de formule III, un composé de formule IV dans laquelle $R^3$ représente un phényle non substitué ou substitué par un méthyle ou un fluor, $R^4$ et $R^5$ sont identiques et représentent un alcoyle inférieur ou ensemble le groupe $-CH_2-O-CH_2-$ , p représente le nombre 0 ou 1 et $R^6$ représente un méthyle, un fluor ou un di-alcoyle inférieur-amino.

**4.** Procédé selon l'une des revendications 1 à 3, caractérise' en ce qu'on utilise comme coordinat de diphosphine $L^1$ , dans un composé de formule III, la (R)-(6,6'-diméthyl-2,2'-biphénylylène)bis-(diphénylphosphine).

**5.** Procédé selon la revendication 1 ou 2, caractérisé en ce qu'on utilise comme coordinat de diphosphine chiral $L^1$ , dans un composé de formule III, la (R)-(1,1'-binaphtyl-2,2'-diylbis(diphénylphosphine).

**6.** Procédé selon l'une des revendications 1 à 5, caractérisé en ce qu'on utilise un composé de départ de formule II , dans laquelle $R^1$ et $R^2$ représentent un éthyle et R représente un radical de formule a) , dans laquelle n vaut zéro.

**7.** Procédé selon l'une des revendications 1 à 6, caractérisé en ce qu'on utilise comme triarylphosphine achirale une triarylphosphine dans laquelle les trois radicaux aryle sont identiques.

**8.** Procédé selon la revendication 7, caractérisé en ce qu'on utilise comme triarylphosphine achirale la triphénylphosphine.

**9.** Complexes constitués d'un complexe de rhodium-diphosphine et d'une triarylphosphine achirale, de préférence la triphénylphosphine.